# EUROPÄISCHE PATENTSCHRIFT

(11) **EP 0 840 792 B1**
(45) Veröffentlichungstag und Bekanntmachung des Hinweises auf die Patenterteilung: **27.02.2002**
(21) Anmeldenummer: 96925698.1
(22) Anmeldetag: 10.07.1996
(51) Int. Cl.: C12N 15/60, C12N 9/88, C12N 1/19, C12P 13/00

(54) **(S)-HYDROXYNITRILLYASE AUS HEVEA BRASILIENSIS**
(S)-HYDROXYNITRILLYASE FROM HEVEA BRASILIENSIS
(S)-HYDROXYNITRILLYASE EXTRAITE D'HEVEA BRASILIENSIS

(30) Priorität: 12.07.1995 AT 118295
(43) Veröffentlichungstag der Anmeldung: 13.05.1998
(73) Patentinhaber: DSM Fine Chemicals Austria Nfg GmbH & Co KG, 4021 Linz (AT)
(72) Erfinder: HASSLACHER, Meinhard, A-8113 St. Oswald (AT); SCHALL, Michael, A-8010 Graz (AT); SCHWAB, Helmut, A-8043 Graz (AT); HAYN, Elfriede Marianne, A-8010 Graz (AT); KOHLWEIN, Sepp, A-8010 Graz (AT); GRIENGL, Herfried, A-8047 Graz (AT)
(74) Vertreter: Klostermann, Ingrid
(86) Internationale Anmeldenummer: EP9603010
(87) Internationale Veröffentlichungsnummer: WO9703204

(56) Entgegenhaltungen:
- EP-A- 0 539 767
- ARCH. BIOCHEM. BIOPHYS., Bd. 311, 1993, Seiten 496-502, XP002022207 J. HUGHES ET AL.: "Purification, characterization, and cloning of alpha-hydroxynitrile lyase from cassava (Manihot esculenta Crantz)"
- PHYSIOL. PLANT., Bd. 75, 1989, Seiten 97-101, XP002022208 D. SELMAR ET AL.: "Alpha-hydroxynitrile lyase in Hevea brasiliensis and its significance for rapid cyanogenesis" in der Anmeldung erwähnt
- J. BIOL. CHEM., Bd. 271, 1996, Seiten 5884-5891, XP002022209 M. HASSLACHER ET AL.: "Molecular cloning of the full-length cDNA of (S)-hydroxynitrile lyase from Hevea brasiliensis"

## Beschreibung

Die Durchführung chemischer Reaktionen unter Zuhilfenahme biologischer Katalysatoren erlangt zunehmend Bedeutung, vor allem in solchen Anwendungsbereichen, in denen die bei Enzymen häufig ausgeprägte Eigenschaft, bei Reaktionen mit chiralen oder prochiralen Komponenten bevorzugt eines der beiden Enantiomeren umzusetzen, genutzt werden kann.

Eines der verwendeten Enzyme ist die (S)-Hydroxynitrillyase (Hnl) aus *Hevea brasiliensis*, welche neben der Bildung aromatischer auch die Bildung aliphatischer (S)-Cyanhydrine aus den entsprechenden Aldehyden beziehungsweise Ketonen mit HCN oder HCN-Donoren katalysiert (EP-A-0 632 130). Dies ist insoferne von Bedeutung, als mit anderen (S)-Hydroxynitrillyasen, wie beispielsweise jener aus *Sorghum bicolor* keine aliphatischen (S)-Cyanhydrine hergestellt werden können (Tetrahedron Letters, **31:** 1249-1252, 1990).

Die bisher bekannte Hnl wird aus den Blättern der *Hevea brasiliensis* gemäß Selmar (Physiologia plantarum **75**: 97-101, 1989) hergestellt und weist eine molekulare Masse von **46** kDa auf (J.E. Poulton in: Cyanide Compounds in Biology [Ciba Foundation Symposium 140), pp 67-91, 1988). Allerdings ist das so isolierte Enzym nicht rein genug, um spezifische Anti-Hnl Antikörper zu gewinnen oder Aminosäuresequenzen des Hnl Proteins zu ermitteln. Alle Versuche, reines HNL-Enzym mittels anderer üblicher chromatographischer Reinigungsschritte zu isolieren, schlugen fehl. Es konnte bei allen Versuchen, das Enzym mittels lonenaustausch-Chromatographie durch Natriumchlorid-Gradienten-Elution in reiner Form zu gewinnen keine Hnl-Aktivität im Säuleneluat gefunden werden. Dies gelang erst, nachdem anstelle des sonst üblichen Natriumchlorid-Gradienten Ammoniumsulfat zur Elution verwendet wurde. Gegenstand der Erfindung ist demnach eine (S)-Hydroxynitrillyase in gereinigter und isolierter Form.
Die so isolierte und gereinigte Hnl besitzt eine molekulare Masse von 30 ± 1 kDa, eine spezifische Aktivität von 19 IU/mg Protein und beinhaltet folgende Aminosäure-Teilsequenzen:

Teilsequenz 1: ...-leu-met-glu-val-phe-pro-...
Teilsequenz 2: ...-gly-ser-leu-phe-gln-asn-...
Teilsequenz 3: ...-glu-ile-ala-glu-ile-leu-gln-glu-val-ala

Dadurch war es in weiterer Folge möglich, nach reverser Transkription von mRNA aus *Hevea brasiliensis* eine cDNA Kopie des *hnl*-Gens zu klonieren, die folgende Nukleotidsequenz aufweist, wobei die davon für das Hnl Protein abgeleitete Aminosäuresequenz darunter angegeben ist und die aus dem Hnl Protein ermittelten Teilsequenzen durch unterstreichen gekennzeichnet sind.

(-43)G AAG AGC ACA TAT CGA TAG TAA AGA GTA AGA TAT CAT CAG AAA

1/1 31/11
ATG GCA TTC GCT CAT TTT GTT CTT ATT CAT ACC ATA TGC CAC GGT GCA TGG ATT TGG CAC
Met ala phe ala his phe val leu ile his thr ile cys his gly ala trp ile trp his

61/21 91/31
AAG CTC AAA CCC CTC CTT GAG GCA CTT GGC CAC AAG GTT ACT GCA CTG GAC CTT GCA GCA
lys leu lys pro leu leu glu ala leu gly his lys val thr ala leu asp leu ala ala

121/41 151/51
AGC GGC GTT GAC CCA AGG CAA ATT GAG GAG ATT GGC TCA TTT GAT GAG TAT TCT GAA CCC
ser gly val asp pro arg gln ile glu glu ile gly ser phe asp glu tyr ser glu pro

181/61 211/71
TTG TTG ACG TTC TTG GAG GCA CTC CCT CCA GGG GAA AAG GTG ATT CTG GTT GGC GAG AGC
leu leu thr phe leu glu ala leu pro pro gly glu lys val ile leu val gly glu ser

241/81 271/91
TGT GGA GGA CTC AAT ATA GCA ATT GCT GCT GAT AAA TAC TGT GAA AAG ATT GCA GCT GCT
cys gly gly leu asn ile ala ile ala ala asp lys tyr cys glu lys ile ala ala ala

301/101 331/111
GTT TTC CAC AAT TCA GTA TTG CCA GAC ACC GAG CAC TGC CCA TCT TAC GTC GTG GAT AAG
val phe his asn ser val leu pro asp thr glu his cys pro ser tyr val val asp lys

361/121 391/131
CTC ATG GAG GTG TTT CCC GAC TGG AAA GAC ACC ACG TAT TTT ACG TAC ACT AAA GAT GGC
leu met glu val phe pro asp trp lys asp thr thr tyr phe thr tyr thr lys asp gly

421/141 451/151
AAG GAG ATA ACT GGA TTG AAA CTG GGC TTC ACG CTT CTG AGG GAA AAT TTA TAT ACC CTT
lys glu ile thr gly leu lys leu gly phe thr leu leu arg glu asn leu tyr thr leu

481/161 511/171
TGC GGT CCT GAG GAA TAT GAA CTG GCG AAG ATG TTG ACA AGG AAG GGA TCA TTA TTT CAA
cys gly pro glu glu tyr glu leu ala lys met leu thr arg lys gly ser leu phe gln

541/181 571/191
AAT ATT TTA GCT AAG CGA CCA TTC TTC ACT AAG GAA GGT TAC GGA TCG ATT AAG AAA ATT
asn ile leu ala lys arg pro phe phe thr lys glu gly tyr gly ser ile lys lys ile

601/201 631/211
TAT GTG TGG ACC GAC CAA GAC GAA ATA TTT TTA CCT GAA TTT CAA CTC TGG CAA ATA GAA
tyr val trp thr asp gln asp glu ile phe leu pro glu phe gln leu trp gln ile glu

661/221 691/231
AAC TAT AAA CCA GAC AAG GTT TAT AAG GTC GAA GGT GGA GAT CAT AAA TTG CAG CTT ACA
asn tyr lys pro asp lys val tyr lys val glu gly gly asp his lys leu gln leu thr

721/241 751/251
AAG ACT AAG GAG ATC GCT GAA ATT CTC CAA GAG GTG GCT GAT ACC TAT AAT TGA CTT CTT
lys thr lys glu ile ala glu ile leu gln glu val ala asp thr tyr asn OPA

TGAGGCTTTTGTTACTATTAAGTATGGAGCAACTATGAGTTAATAATCTCACATTTTCAAGTGGAATTAAGTTGTG
CTAAAATAAAGTTGTTTATTGTGTTGTAATTTTTTTTCATTTGAAGTGGACAGTCTCGCACGCTTTCGAGACTCTTT
ATTTATATATATAATGTAAGTGTGTATTTAAGGAAAGCTACCCCTATTGTGTAGCTTATCATGCTTTTCTTTGAATCA
AATAAATAAAACTTATTT

Die cDNA enthält den gesamten kodierenden Bereich des *hnl*-Gens mit einem offenen Leserahmen für ein Polypeptid von **257** Aminosäuren. Die molekulare Masse wurde aus der von der ermittelten DNA-Sequenz abgeleiteten Aminosäuresequenz des kodierenden Proteins mit **29.227** Da errechnet.
Ein weiterer Gegenstand der Erfindung ist demnach eine DNA-Sequenz, die für (S)-Hydroxynitrillyase kodiert oder mit dieser Sequenz im für Hydroxynitrillyase kodierenden Bereich zu über 85 % identisch ist. Sie wurde durch reverse Transkription aus mRNA gewonnen. Die cDNA stellt die Basis für die Gewinnung
von Enzympräparationen durch heterologe Expression in verschiedenen Wirtsorganismen dar.
Die vorliegende Erfindung betrifft demnach weiters rekombinante Proteine, herstellbar durch heterologe Expression des *hnl*-Gens (cDNA) aus *Hevea brasiliensis* in geeigneten Mikroorganismen, vorzugsweise in eukaryotischen Mikroorganismen.

Es hat sich insbesondere gezeigt, daß sich rekombinantes Hnl Protein, das durch heterologe Expression des *Hevea brasiliensis hnl*-Gens (cDNA) in eukaryotischen Mikroorganismen, wie etwa in *Saccharomyces cerevisiae* oder *Pichia pastoris* hergestellt wurde, deutlich vom natürlichen, aus der Pflanze *Hevea brasiliensis* isolierten Hnl Protein unterscheidet. Das wesentliche Charakteristikum ist, daß die spezifische Aktivität von solchem rekombinanten Hnl Protein deutlich höher ist als die spezifische Aktivität des gereinigten natürlichen Proteins aus *Hevea brasiliensis*. Die Unterschiede manifestieren sich auch im elektrophoretischen Verhalten der Proteine. Sowohl bei der isoelektrischen Fokussierung als auch bei Trennung in nativen Polyacrylamid Gelen werden die Proteinbanden von gereinigten rekombinanten und natürlichen Hnl Proteinen bei unterschiedlichen Positionen aufgefunden. Es wird angenommen, daß dieses unterschiedliche Verhalten auf in der Pflanze und in den Mikroorganismen nicht ident ablaufende post-translationale Modifikationsprozesse zurückzuführen ist und daß die höhere spezifische Aktivität des rekombinanten Hnl Proteins auf in eukaryotischen Mikroorganismen anders modifizierte Proteinmoleküle zurückzuführen ist.

### Beispiel 1

### Gewinnung von reinem Hnl Protein aus Hevea brasiliensis

Portionsweise wurden je 8 g Blätter von *Hevea brasiliensis* (gelagert bei -20 °C in 80 ml 20 mM Kaliumphosphat -Puffer pH 6.5 mit einem Omnimixer (10000 U/min, 1.5 min) unter Eiskühlung homogenisiert. Der erhaltene Extrakt wurde 1 Stunde bei 4 °C gehalten und danach zum Abtrennen der groben Zellbestandteile durch Damen-Nylon-Strümpfe filtriert. Das Retentat wurde nochmals mit 20 mM Kaliumphosphat-Puffer pH 6.5 gewaschen. Kleine Partikeln wurden durch Zentrifugation (40 min, 18000 Upm) entfernt. Dieser so erhaltene Proteinrohextrakt wurde in folgender Sequenz chromatographisch gereinigt:

### Ionenaustauschchromatographie

Eine QAE-Sepharose F.F. Säule (XK 16, 21 cm, Pharmacia, Uppsala, Schweden) wurde mit Startpuffer I (10 mM Histidin-Schwefelsäure pH 6.5, 10 % Sorbit) äquilibriert. Nach der Probenauftragung wurde die Säule mit mindestens 50 ml Start-Puffer gewaschen. Zur Elution wurde ein linearer Gradient von 0 bis 0.6 M (NH₄)₂SO₄ in Startpuffer I verwendet. Fraktionen (10 ml) wurden gesammelt und auf Hnl Aktivität getestet, und Fraktionen mit Hnl Aktivität gepoolt.

### Hydrophobe-Interaktions-Chromatographie

Eine Säule (XK 26, 11 cm), gepackt mit Phenyl-Sepharose low subsitution, (Pharmacia) wurde mit Startpuffer H (0.65 M (NH₄)₂SO₄ in 0.1 M Kaliumphosphat-Puffer pH 6.0) äquilibriert. Die vereinigten Hnl Fraktionen der lonenaustauschchromatographie wurden auf 25 % (NH₄)₂SO₄ Sättigung eingestellt und aufgetragen. Dann wurde die Säule mit 200 ml Start-Puffer gewaschen und anschließend mit einem fallenden linearen (NH₄)₂SO₄ Gradienten (in 0.1 M Kaliumphosphat, pH 6.0) eluiert. 10 ml Fraktionen wurden gesammelt, auf Hnl Aktivität getestet, und Fraktionen mit Hnl Aktivität gepoolt.

### Ausschlußchromatographie

Eine Biogel P150 Säule (26 x 34 cm, Biorad, Hercules, California) wurde mit 100 mM Kaliumphosphat-Puffer pH 6.5 äquilibriert. Das Volumen der Enzymlösung wurde vor dem Auftragen durch Ultrafiltration (Ausschlußgrenze 10000 Da, Amicon Inc., Beverly, MA) reduziert. Die Elution erfolgte mit 100 mM Kaliumphosphat-Puffer pH 6.5 bei Raumtemperatur, Fraktionen (6 ml) wurden gesammelt. Das so gereinigte Hnl Protein zeigte in der SDS-Polyacrylamid Gelelektrophorese nur mehr eine einzige Bande bei einem Molekulargewicht von 30 ± 1 kDa und hatte eine spezifische Aktivität von 19 IU/mg.

### Test auf Hnl Aktivität

Die Hnl-Enzymaktivität wurde über die Bildung von Benzaldehyd aus racemischem Mandelsäurenitril bei 25 °C und pH 5.0 verfolgt. 50 µl Enzymlösung wurden mit 900 µl 50 mM Natriumcitrat-Puffer pH 5.0 gemischt und der Aktivitätstest durch Zugabe von 100 µl Substratlösung (37.5 mM Mandelsäurenitril in 10 mM Natriumcitrat-Puffer pH3.5, täglich frisch hergestellt) gestartet. Es wurde die Zunahme der Absorption bei 280 nm (Messung gegen Substratlösung ohne Enzym) 5 min lang verfolgt. 1 IU entspricht der Enzymmenge, die die Umsetzung von 1 µmol Benzaldehyd pro Minute aus Mandelsäurenitril unter den gegebenen Bedingungen katalysiert.

### Beispiel 2

### Herstellen einer Expressions-cDNA Genbank von Hevea brasiliensis

mRNA wurde aus jungen Blättern eines 10 Jahre alten Baumes der Gattung *Hevea brasiliensis* aus dem botanischen Garten der Universität Graz nach Standardmethoden präpariert (Ausubel et al., Current Protocols in Molecular Biology, Green Publishing Associates and Wiley Interscience, New York, 1990). Die Herstellung der cDNA Genbank erfolgte unter Verwendung und nach den Instruktionen der Unterlagen des "Zap-cDNA Synthesis Kit" sowie des "Gigapack II Gold Packaging Extract" (Stratagene Cloning Systems, La Jolla, CA, U.S.A.).

### Beispiel 3

### Isolierung eines rekombinanten Plasmides, dessen exprimiertes Protein mit Antiserum gegen Hevea brasiliensis Hydroxynitrillyase immunologisch wechselwirkt

Ca. 100 000 Phagen der cDNA Genbank wurden in einem Immunscreening nach Standardmethoden unter Verwendung eines polyklonalen Anti-Hnl-Antiserums (Kaninchen) untersucht. Die Visualisierung des spezifisch gebundenen Antikörpers erfolgte mit einem auf alkalischer Phosphatase und dem chromogenen Substrat NBT (Nitroblue Tetrazolium) / X-Phosphat(5-Bromo-4-chloro-3-indolyl phosphat; 4-Toluidin Salz) basierenden Detektionssystem (Boehringer Mannheim Biochemica, Mannheim, BRD). Von einem erhaltenen positiven Klon wurde das Insert aus der Phagen-DNA nach dem Protokoll "In vivo Excision of pBluescript from Uni-Zap XR"(Stratagene Cloning Systems, La Jolla, CA, U.S.A.) in das entsprechende rekombinante Plasmid umgesetzt (pHNL-100). Die Größe des cDNA Inserts betrug 1100 bp. pHNL-100 wurde in *E.coli* SOLR (Stratagene Cloning Systems, La Jolla, CA, U.S.A.) transformiert. Durch Induktion mit 1 mM IPTG konnte ein mit dem Anti-Hnl Antiserum immunreaktives LacZ-Hnl Fusionsprotein mit einem Molekulargewicht von etwa 30 - 32 kDa nachgewiesen, sowie in cytosolischen Proteinfraktionen Hydroxynitrillyase Aktvität von 0,035 IU/mg Protein detektiert werden. Sämtliche molekularbiologischen Arbeitstechniken wurden aus Ausubel et al. (Current Protocols in Molecular Biology, Greene Publishing Associates and Wiley-Intersience, New York, 1990) entnommen.

### Beispiel 4

### Sequenzierung des cDNA Fragmentes aus Hevea brasiliensis und Klonierung der Vollängen cDNA mit PCR-Methoden

Die DNA-Sequenzierung wurde nach der Kettenterminations Methode von Sanger et al. (Sanger et al., PNAS, 74:5463-5467, 1977) unter Verwendung des "DyeDeoxy Terminator Cycle Sequencing Kit" (Applied Biosystems Inc., Foster City, CA, U.S.A.) und eines automatischen "DNA Sequencer 373A" (Applied Biosystems) durchgeführt. Aus ersten Sequenzdaten konnte ersehen werden, daß im Plasmid pHNL-100 ein unvollständiges cDNA Insert enthalten ist. Der fehlende Teil im 5'-Bereich wurde wie nachfolgend beschrieben ergänzt: Von der *H. brasiliensis* cDNA Genbank wurde Phagen-DNA isoliert und als Matrize für eine zweistufige PCR mit zwei genspezifischen Primern (Primer 1: CCTCCAAGAACGTCAACAAG; Primer 2: CATCAAATGAGCCAATCTCC) und einem Vektor-spezifischen Primer (T3: AATTAACCCTCACTAAAGGG) eingesetzt. PCR Zyklus1: nur Primer 1 und 40 mg cDNA-Genbank (*H. brasiliensis*) DNA; PCR Zyklus 2: Primer 2 und T3 und 1/10 Volumen aus PCR Zyklus1 als DNA Matrize. Die erhaltene DNA aus PCR Zyklus 2 wurde mit *Eco*RI und Styl geschnitten und das resultierende Fragment in den entsprechenden Bereich des Plasmids pHNL-100 (*Eco*RI und Styl geschnitten) einkloniert. Das Insert im resultierenden Konstrukt mit der Bezeichnung pHNL-101 wurde komplett sequenziert.
Eine Analyse der DNA-Sequenz ergab einen offenen Leserahmen beginnend mit ATG bei Position 1 und endend mit einem TGA Stoppkodon bei Position 772, der für ein Protein von **29.227** Da kodiert. Die Größe dieses Proteins korreliert mit dem ermittelten Molekulargewicht der aus Blättern von *H. brasiliensis* isolierten Hydroxynitril Lyase (Hnl). Die für dieses Protein kodierende DNA-Sequenz wurde als *hnl* Gen (cDNA) von *H. brasiliensis* festgelegt.

### Beispiel 5

### Herstellung von Hydroxynitrillyase Präparationen durch Überexpression des H. brasiliensis hnl Gens (cDNA) in Escherichia coli

Aus Gründen der Kloniertechnik wurden neue Restriktionsschnittstellen, *Nco*I im Bereich des Startkodons (Position 1) und *Hind*III nach dem Stopkodon (Position 783), unter Verwendung von Standard PCR Techniken eingeführt (Ersatz der entsprechenden Regionen durch die PCR Fragmente). Als Matrize diente in beiden Fällen DNA des Plasmides pHNL-101.
3' Bereich: PCR mit Primer P51-HX (CCGCTCGAGAAGCTTCAAAGAAGTCAATTATAG) und Primer P51-3.2 (CACGCTTCTGAGGGAAAAT), Schneiden der DNA aus der PCR mit *Xho*I und *Cel*II und einklonieren des Fragments in das Plasmid pHNL-101 *(Xhol* und *Cel*II geschnitten). Das entstandene Plasmid wurde pHNL-102 benannt.
5' Bereich: PCR mit Primer P51-EN (GGAATTCCATGGCATTCGCTCATTTT) und Primer P51-3.1A (CCTCCAAGAACGTCAACAAG), Schneiden der DNA aus der PCR mit *Eco*RI und Styl und einklonieren des Fragments in das Plasmid pHNL-102 (*Eco*RI und Styl geschnitten). Das entstandene Plasmid mit der Bezeichnung pHNL-103 wurde durch Sequenzierung überprüft.
Das *Ncol - Hind*III Fragment aus pHNL-103 wurde im letzten Schritt in den *E.coli* Expressionsvektor pSE420 (Invitrogen Corp., San Diego, CA, U.S.A.) kloniert. Das erhaltene Plasmid pHNL-200 wurde durch Sequenzierung überprüft und in den *E.coli* Stamm Top10' (Invitrogen Corp., San Diego, CA, U.S.A.) transformiert.
Eine entsprechende Transformante wurde in 100 ml 2xYT Medium (10g/l NaCI, 10 g/l Hefe Extrakt, 16 g/l Bactotryptone), supplementiert mit 100mg/l Ampicillin-Na Salz, in einem Schikanen-Schüttelkolben bei 37 °C und 160 Upm bis zu einer optischen Dichte von OD₆₀₀ = 0.5 gezüchtet. Die Proteinproduktion wurde durch Zugabe von 1mM IPTG (Isopropyl-β-D-thiogalaktopyranosid) induziert und die Kultur nach Zusatz von 1% Glukose für 3 h unter den gleichen Bedingungen weitergeführt. Die Zellen wurden anschließend durch Zentrifugation geerntet und in 4ml Aufschlußpuffer (50 mM Kaliumphosphat pH 7.4, 1mM EDTA [Ethylendiamintetraacetat], 1mM PMSF [Phenylmethylsulfonylfluorid], 5% Glycerin) aufgenommen. Der Aufschluß der Zellen erfolgte unter Eiskühlung mit einem Ultraschalldesintegrator (Labsonic 2000, Fa. Braun) mit 45 Watt über 3 min. Die Aufschlußlösung wurde durch Zentrifugation für 15 min bei 27000 g und 4 °C in einem Sorvall SS-34 Rotor und einer Sorvall RC-5 Zentrifuge (DuPont Company, Wilmington, Delaware, U.S.A.) in lösliche und unlösliche Bestandteile aufgetrennt. Die lösliche Fraktion enthielt Protein mit Hydroxynitrillyase Aktivität (0.15 U/mg Protein). Eine Analyse der Proteine in den löslichen und unlöslichen Fraktionen durch SDS Polyacrylamid Gelelektrophorese und Western Blotting ergab, daß nur etwa 1% des gesamten produzierten heterologen immunreaktiven Hnl Proteins in der aktiven löslichen Fraktion vorlag, während 99% des immunreaktiven Hnl Proteins in der unlöslichen Fraktion in Form von nicht aktiven "Einschlußkörpern" zu finden waren.
Proteine der unlöslichen Fraktion wurden durch Zugabe von 20 ml Denaturierungspuffer (3.5 M Harnstoff, 0.1M Tris; pH 8.0) solubilisiert und die unlöslichen Bestandteile durch Zentrifugation (15 min bei 27000 g bei 4 °C) entfernt. Die erhaltene Proteinlösung wurde in mehreren Stufen zuerst gegen Puffer1 (50 mM Kaliumphosphat pH 7.4, 1mM EDTA, 1M Harnstoff) und 5 mal gegen Puffer 2 (50 mM Kaliumphosphat pH 7.4, 1mM EDTA) bei 4 °C dialysiert. Die so erhaltene solubilisierte und renaturierte Proteinpräparation zeigte eine spezifische Hnl Aktivität von 0.8 bis 1.4 IU/mg Protein.

### Beispiel 6

### Herstellung von Hydroxynitrillyase Präparationen durch Überexpression des H. brasiliensis hnl Gens (cDNA) in Saccharomyces cerevisiae

Aus kloniertechnischen Gründen wurden im Bereich der *Hind*III Restriktionsschnittstelle des Plasmides pHNL-103 zwei neue Schnittstellen (*Eco*RI, *Bam*HI) durch Ligation des mit *Hind*III linearisierten Plasmides mit dem Adaptor B/E (AGCTTGAATTCGGATCC; AGCTGGATCCGAATTCA) eingeführt. Das entstandene Konstrukt mit der Bezeichnung pHNL-104 wurde durch Sequenzierung überprüft.
Das *hnl* Gen (cDNA) wurde als *Bam*HI Fragment aus dem Plasmid pHNL-104 entnommen und in den mit *Bgl*II linearisierten Hefe-Expressionsvektor pMA91 (Kingsman et al., Methods in Enzymology, 185:329-341, 1990) kloniert. Das resultierende Plasmid mit der Bezeichnung pHNL-300 wurde durch Sequenzierung überprüft und weiter in den *S. cerevisiae* Laborstamm W303 D (Hill et al., Yeast, 2:163-167, 1986) transformiert. Transformanten wurden auf Minimalmedium ohne Leucin (6.7 g/l Yeast Nitrogen Base w/o Amino Acids [Fa. Difco, U.S.A], 20 g/l Glukose, 20 ml/l Aminosäurekonzentrat ohne Leucin [Adenin 1.0 g/l, Methionin 1.0 g/l, Arginin 1.0 g/l, Threonin 15.0 g/l, Histidin 1.0 g/l, Tryptophan 1.0 g/l, Uracil 2.0 g/l, Lysin 11.5 g/l]) gezüchtet. Für die Proteinproduktion wurden die Zellen in einem Schikanenerlenmeyerkolben in 100 ml leucinfreiem Minimalmedium bei 30°C und 150 Upm bis zu einer optischen Dichte von OD600 = 5.0 gezüchtet und durch Zentrifugation geerntet. Die Zellen wurden in 5 ml Aufschlußpuffer (wie bei Beispiel 5) aufgeschlämmt und nach der "Glaskugelmethode" (Ausubel et al., Current Protocols in Molecular Biology, Greene Publishing Associates and Wiley-Intersience, New York, 1990) in einem Merckenschlager (Fa. Braun, Melsungen, BRD) aufgeschlossen. In der löslichen cytosolischen Fraktion konnte eine Hydroxynitrillyaseaktivität von 4.62 IU/mg Protein nachgewiesen werden. Als Kontrolle dienten Hefe-Transformanten mit dem leeren Plasmid pMA91, wo keine cytosolische Hydroxynitrillyaseaktivität nachgewiesen werden konnte. Die so hergestellten wäßrigen Proteinpräparationen wurden bei -20°C gelagert. Eine weitere Möglichkeit für eine dauerhafte Lagerung der Proteinpräparation bestand darin, das Präparat nach Entfernung sämtlicher niedermolekularer Substanzen (Dialyse bei 4°C gegen destilliertes Wasser) bzw. Aufschluß der Hefezellen nach Suspension in Wasser gefrierzutrocknen (Benchtop 3L, VIRTIS Co., Inc., Gardiner, NY, U.S.A.). Die Aktivität einer in Aufschlußpuffer (wie in Beispiel 5) resolubilisierten Enzymprobe blieb dabei nahezu zu 100% erhalten.
Es war auch weiters leicht möglich, ausgehend von den wäßrigen Proteinpräparationen hochgereinigtes rekombinantes Hnl Protein herzustellen. Nach den gleichen Verfahren wie in Beispiel 1 beschrieben, konnte auf einfache Weise eine Enzympräparation, die bei SDS-Polyacrylamid Gelelektrophorese praktisch nur mehr eine Bande bei 30 ± 1 kDa zeigt, gewonnen werden. Solche Präparationen von rekombianter, durch Überexpression in *S.cerevisiae* gewonnener Hnl zeigten spezifische Aktivität von 22 bis 28 IU/mg Protein.

### Beispiel 7

### Herstellung von Hydroxynitrillyase Präparationen durch Überexpression des H. brasiliensis hnl Gens (cDNA) in Pichia pastoris

Das *hnl* Gen (cDNA) wurde als *Eco*RI Fragment aus dem Plasmid pHNL-104 in den mit *Eco*RI linearisierten *P. pastoris* Expressionsvektor pHIL-D2 (Invitrogen Corp., San Diego, CA, U.S.A.) kloniert. Das Konstrukt mit der Bezeichnung pHNL-400 wurde durch Sequenzierung überprüft.
Die Transformation des Wirtstammes GS115 (His4-), Selektion der Histidin Prototrophen und gleichzeitig Methanol-Verwertungs Auxotrophen erfolgte nach den Unterlagen des "*Pichia* Expression Kit" Systems (Invitrogen Corp., San Diego, CA, U.S.A.). Unter 20 positiven Transformanten konnten zwei identifiziert werden, welche die Hydroxynitrillyase in hoher Konzentration intrazellulär produzierten. Die Anzucht der Zellen für die Proteinproduktion erfolgte ebenfalls nach den Protokollen des *"Pichia* Expression Kit". Die durch Zentrifugation geernteten Zellen wurden in Aufschlußpuffer (wie Beispiel 5) zu einer optischen Dichte von OD600 = 50.0 suspendiert und nach der "Glaskugelmethode" (wie bei Beispiel 6) aufgeschlossen. In der löslichen cytosolischen Fraktion konnte Hydroxynitrillyase Aktivität von 16 U/mg Protein detektiert werden. Die so hergestellten Proteinpräparationen konnten in gleicher Weise wie bei Beispiel 6 beschrieben ohne nennenswerte Aktivitätsverluste gelagert werden.
Nach den gleichen Verfahren wie in Beispiel 1 beschrieben, kann auf einfache Weise eine hochgereinigte Enzympräparation gewonnen werden, die bei SDS-Polyacrylamid Gelelektrophorese praktisch nur mehr eine Bande bei 30 ± 1 kDa zeigte. Solche Präparationen von rekombianter, durch Überexpression in *P.pastoris* gewonnener Hnl zeigten spezifische Aktivität im Bereich von 41 bis 46 IU/mg Protein.

### Beispiel 8

### Vergleich der rekombinanten Hnl-Produkte

Gereinigte Hnl-Proteinpräparationen wurden durch isoelektrische Fokussierung mit Gelen 3-9 (Phast System, Pharmacia, Uppsala, Schweden) oder durch native Polyacrylamid Gelelektrophorese (7.5 % Polyacrylamid, Tris-Glycin Puffer pH 8.8) analysiert. Es konnte dabei festgestellt werden, daß unterschiedliche Banden bei den verschiedenen Präparationen zu finden waren.
Bei isoelektrischer Fokussierung zeigte Hnl aus *H.brasiliensis* eine Bande bei einem isoelektrischen Punkt von 4.1. Bei den rekombinanten Proteinen aus *S.cerevisiae* und *P.pastoris* konnten zwei bis drei knapp beisammenliegende Banden gefunden werden, die etwas weiter in Richtung zu basischen Bereichen entsprechenden Positionen lokalisiert waren. Bei rekombinanter Hnl aus *P.pastoris* war ein überwiegender Anteil bei diesen verschobenen Positionen zu finden.
Bei nativer Polyacrylamid Gelelektrophorese zeigten die rekombinanten Proteine aus *S.cerevisiae* und *P.pastoris* eher ähnliches Verhalten. Es konnten jeweils zwei starke eng beisammenliegende Banden, flankiert von zwei schwachen Banden als spezifisch für Hnl Protein durch Western-Blotting (mit polyklonalem anti-Hnl Antiserum) identifiziert werden. Bei Analyse des Proteins aus *H.brasiliensis* konnte im Gegensatz dazu eine etwas diffuse Bande identifiziert werden, die etwas weiter gelaufen war.

### Beispiel 9

### Identifikation von essentiellen, an der Katalyse beteiligten Aminosäuren der Hydroxynitrillyase

Recherchen in Proteindatenbanken (Swissprot, PIR, Genpept) unter Verwendung des Suchmoduls BLAST (Altschul et al., J. Mol. Biol. 215, 403-410, 1990) sowie die Erstellung von "multiplen Alignments" und deren statistische Analyse unter Verwendung von Programmodulen des GCG-Software Paketes (Program Manual for the Wisconsin Package, Version 8, September 1994, Genetics Computer Group, **575** Science Drive, Madison, Wisconsin, USA 53711 ) führten zu folgender Interpretation: Hydroxynitrillyase aus *Hevea brasiliensis* dürfte einer großen Gruppe strukturell verwandter Proteine des "α/β Hydrolase fold" Typs (Ollis et al., Protein Engineering, Vol. 5, 197-211, 1992) angehören. Neben einer charakteristischen Tertiärfaltung besitzen diese Proteine eine sogenannte katalytische Triade mit Asp od. Ser od. Cys als nukleophilen Teil der Triade sowie Asp/Glu und His. Um zu beweisen, daß diese aus Computervorhersagen ermittelten Aminosäuren auch an der katalytischen Aktivität der Hydroxynitrillyase beteiligt sind (Glu79, Ser80, Cys81, Asp207 und His235), wurden mutante Proteine der Hydroxynitrillyase mit jeweils der Aminosäure Alanin an den Positionen 79, 80, 207, 235 sowie Serin an Position 81 hergestellt. Die Mutationen wurden auf Ebene des Plasmides pHNL-104 (siehe Beispiel 6) unter Anwendung von Standard-PCR Methoden eingeführt. Für die Veränderung der Aminosäure-Positionen 79, 80, und 81 wurde jeweils ein mutanter, antisense Endprimer, der auch die für die Subklonierung notwendige, genspezifische Restriktionsschnittstelle *Mun*I überlappt, und ein vektorspezifischer Primer (T3) verwendet (Ausubel et al., Current Protocols in Molecular Biology, Vol.1 & 2, Greene Publishing Associates and Wiley-Intersience, New York, 1990). Für die Veränderung der Aminosäure-Positionen 207 und 235 kam ein spezielles PCR-Verfahren zur Anwendung. Dabei wurden ein phosphorylierter mutanter Primer in sense Richtung, ein genspezifischer Primer, der auch die für die Subklonierung notwendige Restriktionsschnittstelle *Ce*/ll überlappt und ein weiterer vektorspezifischer Primer (T7) verwendet (Michael S.F., BioTechniques 16, 410-412, 1994). Die Aufreinigung der PCR Produkte und Subklonierung erfolgte nach Standardmethoden. Die resultierenden mutanten *hnl* Gene (cDNA) in den Plasmiden pHNL-105 - pHNL-109 wurden in das Expressionplasmid pMA91 kloniert (pHNL-302 - pHNL-306) und die mutanten Proteine in *Saccharomyces cerevisiae*, wie unter Beispiel 6 beschrieben, produziert. In der löslichen cytosolischen Fraktion wurde die Hydroxynitrillyase Aktivität bestimmt mit dem Ergebnis, daß jede der 5 durchgeführten Mutationen zur vollständigen Inaktivierung der Enzymakivität führte. Eine Beteiligung dieser Aminosäuren an der direkten enzymatischen Katalyse kann daraus abgeleitet werden. Der globale Erhalt der Proteinstruktur bei den mutanten Proteinen im Vergleich zum nichtmutierten Protein durch nahezu gleiches Laufverhalten bei isoelektrisches Fokussierung oder nativer Polyacrylamid Gelelektrophorese verifiziert (Ausubel et al., Current Protocols in Molecular Biology, Vol.1 & 2, Greene Publishing Associates and Wiley-Intersience, New York, 1990).

## Patentansprüche

1. (S)-Hydroxynitrillyase, welche die von der folgenden DNA-Sequenz SEQ ID NO 1 abgeleitete Aminosäuresequenz SEQ ID NO 2 aufweist oder mit dieser zumindestens 80 % identisch ist.

2. DNA-Sequenz, die für (S)-Hydroxynitrillyase gemäß Anspruch 1 kodiert oder mit dieser Sequenz im für Hydroxynitrillyase kodierenden Bereich zu über 85 % identisch ist.

3. DNA-Sequenz gemäß Anspruch 2, welche die für (S)-Hydroxynitrillyase-Aktivität notwendigen Sequenzen umfaßt.

4. Vektor, der eine DNA-Sequenz gemäß einem der Ansprüche 2 oder 3 enthält.

5. Wirtszellen enthaltend eine DNA-Sequenz gemäß einem der Ansprüche 2 oder 3, die mittels eines Vektors eingebracht wurde.

6. Wirtszellen gemäß Anspruch 5, **dadurch gekennzeichnet, daß** sie Zellen von Mikroorganismen sind.

7. Wirtszellen gemäß Anspruch 5 oder 6, **dadurch gekennzeichnet, daß** sie *Saccharomyces cerevisiae* oder *Pichia pastoris* entstammen.

8. Rekombinantes Protein, herstellbar durch heterologe Expression einer DNA Sequenz gemäß einem der Ansprüche 2 oder 3 in einer Wirtszelle gemäß einem der Ansprüche 5 bis 7.

9. Verfahren zur Herstellung eines Proteins mit (S)-Hydroxynitrillyase-Aktivität, **dadurch gekennzeichnet, daß** Wirtszellen nach einem der Ansprüche 5 bis 7 kultiviert werden und das gebildete Protein isoliert wird.

10. Verwendung eines rekombinanten Proteins gemäß Anspruch 8 zur Herstellung von (S)-Cyanhydrinen.

## Claims

1. (S)-Hydroxy-nitrile-lyase according to Claim 1, which has the amino-acid sequence SEQ ID NO 2 deduced from the following DNA sequence SEQ ID NO 1, or is at least 80% identical with this.

2. DNA sequence which codes for (S)-hydroxy-nitrile-lyase according to Claim 1, or is more than 85% identical with this sequence in the region coding for hydroxy-nitrile-lyase.

3. DNA sequence according to Claim 2, which comprises the sequences necessary for (S)-hydroxy-nitrile-lyase activity.

4. Vector which comprises a DNA sequence according to either of Claims 2 or 3.

5. Host cells comprising a DNA sequence according to either of Claims 2 or 3, which has been introduced by means of a vector.

6. Host cells according to Claim 5, **characterized in that** they are microrganism cells.

7. Host cells according to Claim 5 or 6, **characterized in that** they are derived from *Saccharomyces cerevisiae* or *Pichia pastoris.*

8. Recombinant protein which can be prepared by heterologous expression of a DNA sequence according to either of Claims 2 or 3 in a host cell according to any of Claims 5 to 7.

9. Process for producing a protein with (S)-hydroxy-nitrile-lyase activity, **characterized in that** host cells according to any of Claims 5 to 7 are cultivated and the protein which is formed is isolated.

10. Use of a recombinant protein according to Claim 9 for preparing (S)-cyanohydrins.

## Revendications

1. (S)-hydroxynitrillyase, qui présente la séquence d'acides aminés SEQ ID NO 2 dérivée de la séquence d'ADN suivante SEQ ID NO 1 ou qui est identique à celle-ci à au moins 80 %.

2. Séquence d'ADN, qui code la (S)-hydroxynitrillyase selon la revendication 1 ou est identique à cette séquence dans la zone codant l'hydroxynitrillyase à plus de 85 %.

3. Séquence d'ADN selon la revendication 2, qui comprend les séquences nécessaires à une activité de (S)-hydroxynitrillyase.

4. Vecteur qui comprend une séquence d'ADN selon l'une des revendications 2 à 3.

5. Cellules hôtes contenant une séquence d'ADN selon l'une des revendications 2 ou 3, qui ont été introduites à l'aide d'un vecteur.

6. Cellules hôtes selon la revendication 5, **caractérisées en ce qu'**elles sont des cellules de micro-organismes.

7. Cellules hôtes selon la revendication 5 ou 6, **caractérisées en ce qu'**elles proviennent de *Saccharomyces cerevisiae* ou de *Pichia pastoris.*

8. Protéine recombinante, pouvant être fabriquée par expression hétérologue d'une séquence d'ADN selon l'une des revendications 2 ou 3 dans une cellule hôte selon l'une des revendications 5 à 7.

9. Procédé pour fabriquer une protéine dotée d'une activité (S)-hydroxynitrillyase, **caractérisée en ce que** les cellules hôtes sont cultivées selon l'une des revendications 5 à 7 et que la protéine formée est isolée.

10. Utilisation d'une protéine recombinante selon la revendication 8 pour fabriquer des (S)-cyanhydrines.
